# EUROPEAN PATENT APPLICATION

(11) **EP 3 623 690 A1**
(43) Date of publication of application: **18.03.2020**
(21) Application number: 18798865.4
(22) Date of filing: 23.04.2018
(51) Int. Cl.: F21L 4/00, F21V 5/00, F21V 9/14, F21V 9/40, F21V 13/12, F21V 21/30, G02B 5/30, G02B 27/28, G02C 7/12, G02C 11/04, F21Y 115/10

(54) **HEAD-MOUNTED LIGHTING DEVICE**

(30) Priority: 09.05.2017 JP 2017092910
(71) Applicant: Synqroa Co., Ltd., Tokyo 108-0073 (JP)
(72) Inventor: KOYAMA, Mitsuhiro, Tokyo 108-0073 (JP); AYABE, Kaori, Tokyo 108-0073 (JP)
(74) Representative: advotec.
(86) International application number: PCT/JP2018/016432
(87) International publication number: WO 2018/207602

(57) **Abstract**

Provided is a lighting device according to the present invention including: a support portion 3 disposed in front of a forehead of a head of a wearer; a light source unit 10 provided to the support portion 3, the light source unit 10 including a light source which irradiates an object with light, and a condensing lens 13 which condenses the light emitted from the light source; and a transmission member 30 configured to transmit reflected light from the object so as to allow the wearer to visually recognize the reflected light, the transmission member 30 being provided to the support portion 3 and disposed in front of an eye of the wearer, wherein the light source unit 10 includes a first polarizing element which converts emitted light emitted from the light source to linearly polarized light, and the transmission member 30 includes a second polarizing element which is disposed 90° orthogonal to a polarization axis of the first polarizing element.

## Description

### TECHNICAL FIELD

The present invention mainly relates to a head-mounted lighting device which can be used for various medical actions, such as surgery, operations, examinations, or procedures, and more particularly to a head-mounted lighting device which allows such medical actions to be performed without causing any problem.

### BACKGROUND ART

Conventionally, in a medical site which performs dental therapy, medical surgery, operation, examination, or procedure (collectively also referred to as "treatment"), a lighting device has been used which irradiates an affected part of a patient. This lighting device has a special structure so as to prevent the formation of a shadow of a hand or a head of a medical worker, such as a doctor so that this lighting device is also referred to as a shadowless lamp. In addition to the shadowless lamp having such a configuration, for example, a head-mounted (eyeglass-type) lighting device as disclosed in Patent Literature 1 is also known which irradiates an affected part in a state where the lighting device is mounted on a head of a medical worker (the lighting device is mounted so as to be put on ears).

The head-mounted lighting device disclosed in Patent Literature 1 has a structure where light emitting diodes (LEDs) are incorporated in a light emitting unit, and the light emitting unit is mounted on a front end side of each of a pair of temples to be put on the ears. This head-mounted lighting device irradiates the affected part of a patient in a state where the head-mounted lighting device is mounted by being put on the ears of a medical worker. Accordingly, there is no possibility that a shadow of the head of the medical worker is formed so that the affected part can be irradiated in a shadowless state.

### CITATION LIST

### PATENT DOCUMENT

Patent Document 1: Japanese Unexamined Patent Application Publication No. 2002-224156

### SUMMARY OF THE INVENTION

### PROBLEM TO BE SOLVED BY THE INVENTION

With respect to the head-mounted lighting device having the above-mentioned configuration, the affected part is irradiated with light emitted from light emitting parts (light sources) positioned on the outside of both eyes, and a wearer visually recognizes reflected light from the affected part. With such a configuration, the wearer performs treatment on the affected part while grasping a state of the affected part. However, such a head-mounted lighting device has the following various problems.

Light emitted from the light emitting part and irradiating the inside of a human body or an oral cavity generates glare at the affected part of the human body or the oral cavity. This glare becomes dazzling to the eyes of the wearer, thus preventing the wearer from clearly visually recognizing the affected part. It is necessary for the wearer to perform a proper treatment while grasping a condition of the affected part. Accordingly, if such glare is generated, the wearer changes an angle of light by moving his/her head and, then, visually recognizes the affected part again and hence, labor is required for performing proper treatment. This means that proper treatment cannot be performed within short time, and such a situation is a large burden for both a wearer and a patient.

For example, in abdominal surgery for cancer or the like, after a focus of disease is removed, a nerve plexus is peeled away. At this point of operation, glare generated at the boundary portion becomes an obstacle and hence, it is required to perform treatment with great care so as to prevent a nerve from being damaged. Likewise, glare becomes an obstacle in performing suturing of a blood vessel, thus increasing labor and time for the work. Further, in oral cavity therapy, vacuuming is performed while water is discharged for cooling, for example. However, the discharged water becomes a cause of glare so that long time is required for performing proper treatment. Particularly, in performing treatment relating to dental implant, it is necessary to grasp a proper depth by monitoring a gauge. Accordingly, when the water adheres to the gauge so that glare is generated, the gauge is prevented from being easily observed and hence, treatment time may be extended, or a wearer may perform treatment while relying on intuition.

Further, the head-mounted lighting device preferably has a configuration which has a light weight, excellent operability, and excellent handling, which is mountable for long time, and, further, which does not cause a wearer to have eye fatigue. That is, in performing medical-related therapy or the like, which is a delicate treatment, it is preferable for the head-mounted lighting device to have a structure which provides excellent wearing feeling even during long time use, and which can obtain a light spot having proper illuminance and a light field diameter with a proper distance. During the delicate treatment, a wearer attempts to move mostly only a line of sight without moving a neck so as not to cause fatigue. When a light emitting part is fixed to a temple as in the case of a conventional technique, irradiation range does not follow the line of sight when only the line of sight is moved. As the result, the wearer is required to move the neck, and thus becomes fatigued easily.

The present invention has been made based on the above-mentioned problems, and it is an object of the present invention to provide a head-mounted lighting device which allows proper treatment work to be performed within short time when an object is irradiated with light, and various treatment works are performed while reflected light from the object is visually recognized.

### MEANS FOR SOLVING PROBLEM

To achieve the above-mentioned object, a head-mounted lighting device according to the present invention includes: a support portion disposed in front of a forehead of a head of a wearer; a light source unit provided to the support portion, the light source unit including a light source which irradiates an object with light, and a condensing lens which condenses the light emitted from the light source; and a transmission member configured to transmit reflected light from the object so as to allow the wearer to visually recognize the reflected light, the transmission member being provided to the support portion and disposed in front of an eye of the wearer, wherein the light source unit includes a first polarizing element and a first wavelength plate, the first polarizing element converting emitted light from the light source to linearly polarized light, the first wavelength plate converting the light transmitted through the first polarizing element to circularly polarized light or elliptically polarized light, and the transmission member includes a second wavelength plate and a second polarizing element, the second wavelength plate having a phase difference equal to a phase difference of the first wavelength plate, the second polarizing element being disposed so as to cause the light transmitted through the second wavelength plate to be 90° orthogonal to a polarization axis of the first polarizing element.

In the head-mounted lighting device having the above-mentioned configuration, the light emitted from the light source of the light source unit is condensed by the condensing lens and, at the same time, is converted to linearly polarized light, and an object is irradiated with the linearly polarized light. The reflected light from the object forms linearly polarized light. However, when the surface of the object has unevenness, thus not being in a mirror surface state, reflected light is diffused and reflected (scattered and reflected). That is, in a case where the reflected light from the object is diffused and reflected, even when incident light is linearly polarized light, the reflected light of the incident light becomes a non-polarization state. Light in this non-polarization state contains components of specularly reflected light (the components form linearly polarized light). The components of the specularly reflected light are visually recognized as so-called glossiness (glare) which is the glare of the light source. This glossiness becomes an obstacle when various treatments are performed while an object is visually recognized. The transmission member allows transmission of the reflected light so as to allow the wearer to visually recognize the reflected light. The transmission member includes the second polarizing element disposed 90° orthogonal to the polarization axis of the first polarizing element and hence, reflection components which become a cause of glossiness can be removed whereby the wearer can visually recognize only a diffused and reflected light with no glare. In this case, linearly polarized light is converted to circularly polarized light or elliptically polarized light, an object is irradiated with the circularly polarized light or the elliptically polarized light, and time required for transmission of the light and variation in intensity of the light are observed. With such observation, it is possible to obtain information of chirality, which is a difference in a three-dimensional arrangement of molecules. For example, molecules of water in a dynamic state reflect or absorb light corresponding to a wavelength. That is, the state of the surface of water flowing along the surface of an object is equal to the state of a surface having unevenness. Accordingly, water cannot specularly reflect light, thus causing scattered reflection whereby color information of the light is lost. When the reflected light is viewed while water in a dynamic state is impinged on an object, the reflected light is viewed hazily (white opaque state). The reason of such a phenomenon is that color information of the light is lost, thus preventing light from being clearly visually recognized. Light has characteristics that when the light is reflected off a water surface or the like, the light is changed to light having large lateral oscillation. Accordingly, when reflected light is circularly polarized, longitudinal oscillation is extremely reduced, and the light is changed to light having oscillation almost only in the lateral direction. Therefore, when the reflected light is converted to the linearly polarized light after being converted to the circularly polarized light, glare can be reduced even in a state where a substance in a dynamic state, such as water, is on an object.

### EFFECT OF THE INVENTION

According to the head-mounted lighting device of the present invention, it becomes possible to perform a proper treatment work within short time when an object is irradiated with light, and various treatment works are performed while reflected light from the object is visually recognized.

### BRIEF DESCRIPTION OF DRAWINGS

Fig. 1 is a front view showing a first embodiment of a head-mounted lighting device according to the present invention.
Fig. 2 is a plan view of the head-mounted lighting device shown in Figure 1.
Fig. 3 is a side view of the head-mounted lighting device shown in Figure 1.
Fig. 4 is a back view of the head-mounted lighting device shown in Figure 1.
Fig. 5(a) is a perspective view showing a state where a transmission member is flipped up, and Fig. 5(b) is a perspective view showing a state where the transmission member is flipped down.
Fig. 6 is a perspective view showing a state where the head-mounted lighting device shown in Fig. 1 is mounted on the head.
Fig. 7 is a schematic view of paths of light from a plurality of condensing lenses, which are incorporated in a light source unit, and a light field diameter.
Fig. 8 is a schematic view showing a state where a light field is displaced at the time of rotating the light source unit.
Fig. 9 is a view schematically showing a path of light emitted from the light source unit to a point where the light reaches an eye, and is also a view showing a configuration that an object in a static state is irradiated with light to remove glare.
Fig. 10 is a photograph (photocopy of photograph) obtained when a conventional lighting device is used, the photograph showing an inside of an oral cavity where glare is not removed.
Fig. 11 is a photograph (photocopy of photograph) obtained when a lighting device, provided with optical elements having the configuration shown in Fig. 9, is used, the photograph showing the inside of the oral cavity in a state where glare is removed.
Fig. 12 is a view schematically showing the path of light emitted from the light source to the point where the light reaches the eye, and is also a view showing the configuration that an object in a dynamic state is irradiated with light to remove glare.
Fig. 13 is a photograph (photocopy of photograph) obtained when the conventional lighting device is used, the photograph showing the inside of the oral cavity where glare is not removed.
Fig. 14 is a photograph (photocopy of photograph) obtained when a lighting device, provided with optical elements having the configuration shown in Figure 12, is used, the photograph showing the inside of the oral cavity in the state where glare is removed.
Fig. 15 is a view showing a second embodiment of a head-mounted lighting device according to the present invention.

### MODE(S) FOR CARRYING OUT THE INVENTION

Hereinafter, embodiments of a head-mounted lighting device (hereinafter referred to as a "lighting device") according to the present invention are described with reference to attached drawings. An eyeglass-type lighting device which can be put on ears of a wearer is exemplified as the lighting device described in the embodiments hereinafter. The description is made assuming that the lighting device irradiates an affected part (object) of a patient to allow a medical worker to visually recognize the affected part when the medical worker performs various therapy activities in a state of wearing the lighting device.

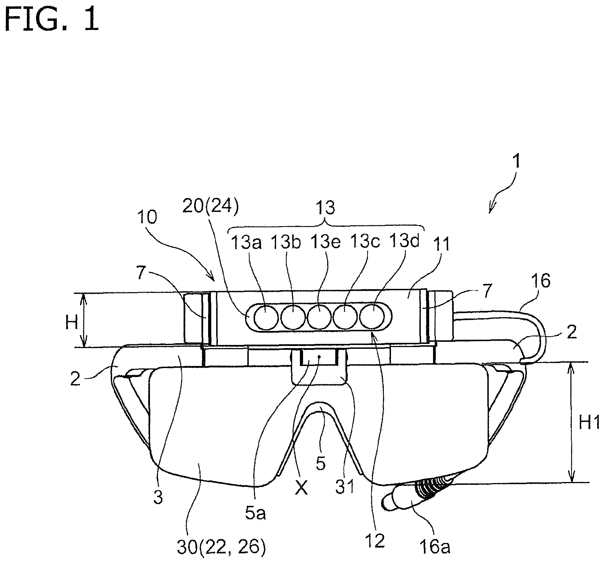

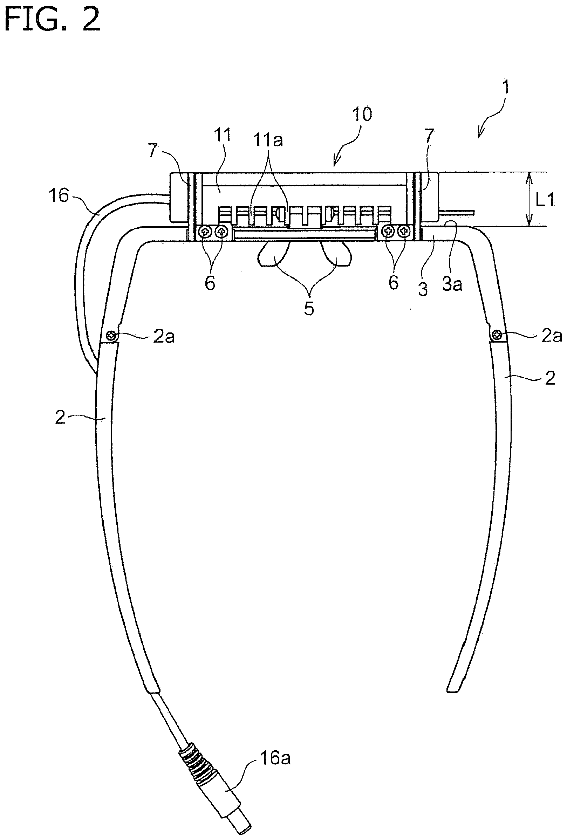

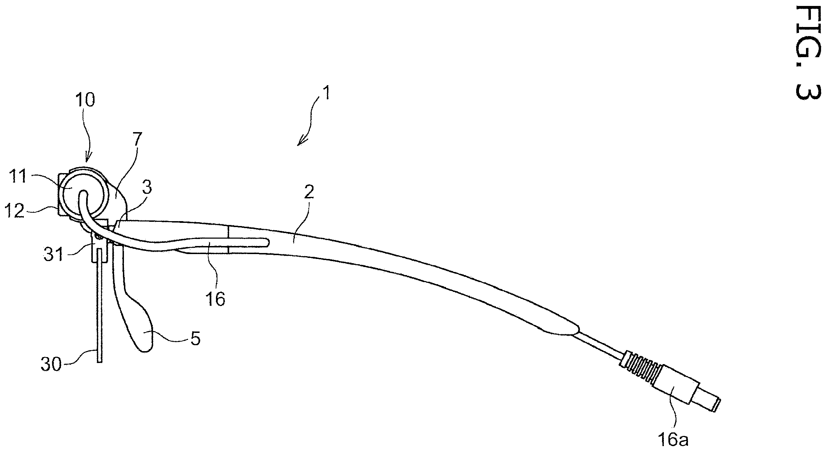

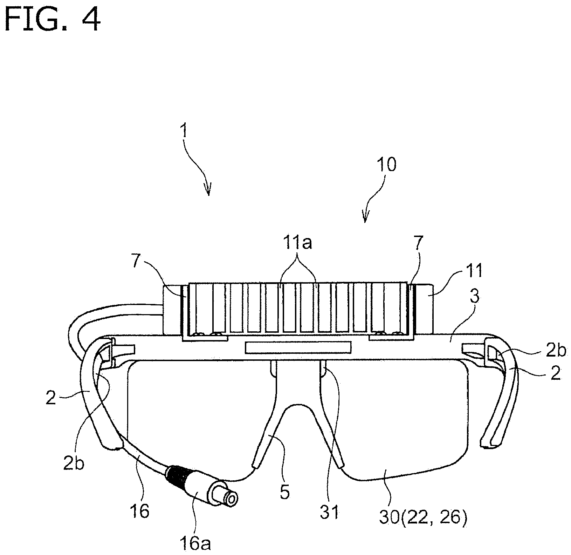

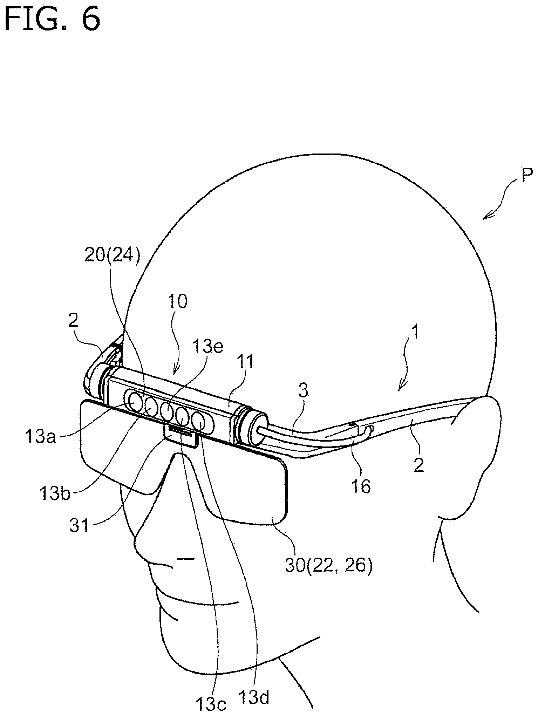

First, an overall configuration of the lighting device according to the first embodiment is described with reference to Fig. 1 to Fig. 8.

A lighting device 1 is of an eyeglass type. The lighting device 1 includes a pair of temples 2, 2 which is put on ears of a medical worker (also referred to as a "wearer") P, and a connection portion (hereinafter, this connection portion is referred to as a "support portion") 3 which connects front end sides of the respective temples with each other. In the same manner as general eyeglasses, the support portion 3 extends in a lateral direction in front of a forehead of the wearer P. In a state where the wearer P wears the lighting device 1, the positional relationship is established where the support portion 3 is disposed above eyes of the wearer P. In this case, the lighting device 1 is configured to have a size which allows a wearer to wear the lighting device 1 not only when the wearer does not wear eyeglasses, but also when the wearer wears eyeglasses.

It is preferable that the temples 2 and the support portion 3 be made of a material having a light weight and flexibility. For example, the temples 2 and the support portion 3 are integrally formed using a synthetic resin, such as polypropylene. A hinge 2a is provided to a distal end side of each temple 2 so that a rear end side of each temple is configured to be foldable. Further, in this embodiment, a recessed groove 2b is formed on an inner side surface of the temple 2. A power cord, which supplies power to a light source unit 10 described later, is embedded into this recessed groove 2b to prevent the power cord from being exposed. With such a configuration, problems, such as a problem that the power cord which gets in the way of performing a therapy activity, comes in view or a problem that the power cord is caught accidentally, are eliminated as much as possible.

The light source unit 10 is provided to an upper side of the support portion 3, and a transmission member 30 is provided to a lower side of the support portion 3. The transmission member 30 allows transmission of reflected light of light, emitted from a light source of the light source unit 10 and irradiating the affected part so as to allow the wearer P to visually recognize the reflected light. Further, a nose pad portion 5 having a substantially inverted Y shape is provided to a lower surface of a center portion of the support portion 3. That is, the light source unit 10 and the transmission member 30 are respectively provided to the upper and lower sides of the support portion 3 extending in the lateral direction (width direction of a face) in a region in front of the forehead.

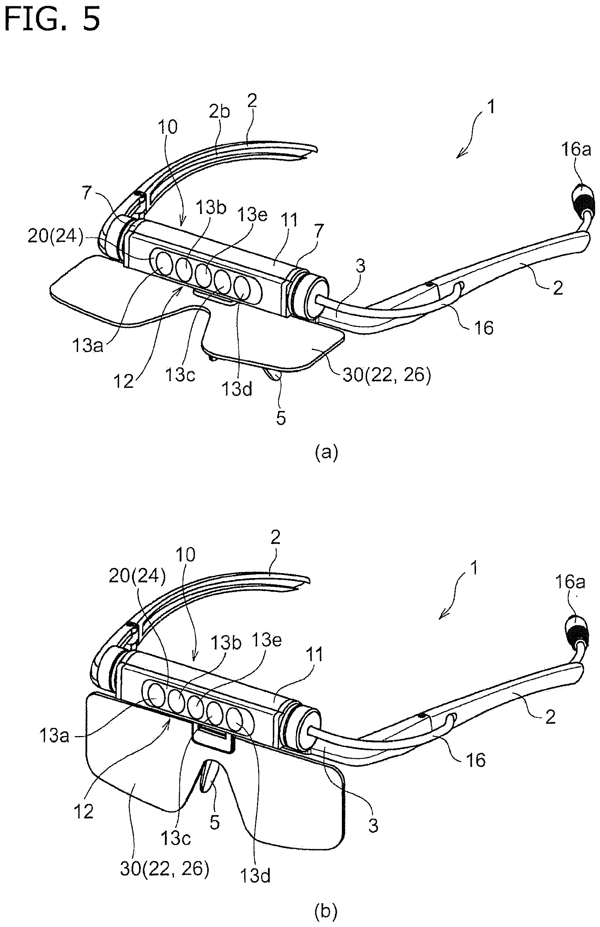

The transmission member 30 in this embodiment is formed of a plate-shaped member having no frame on a periphery thereof so as to prevent the frame from interrupting a line of sight. An upper portion of a center of the transmission member 30 is sandwiched by a flap 31. This flap 31 is fixed to a projecting portion 5a with screws, wherein the projecting portion 5a is provided at a proximal portion of the nose pad portion 5, and protrudes forward. Accordingly, the flap 31 is supported in a rotatable manner in upward and downward directions. With such a configuration, as shown in Figures 5(a) and 5(b), the transmission member 30 is rotatable in the upward and downward directions with respect to the support portion 3 between a horizontal position where the eyes of the wearer P are uncovered and a vertical position where the transmission member 30 covers the eyes of the wearer P. That is, rotating the transmission member 30 to the vertical position allows the wearer P to visually recognize reflected light from the affected part without glare as described later. On the other hand, rotating the transmission member 30 to the horizontal position allows the wearer P to directly and visually recognize a condition of the actual affected part.

The light source unit 10, provided to the upper side of the support portion 3, includes a casing 11 formed into a rod shape to extend along the support portion 3. The light source provided with light emitting elements, such as LEDs, is disposed in the casing 11, for example. The casing 11 has a substantially cylindrical shape where an irradiation side of the casing has a flat shape. To prevent the casing 11 from entering the field of vision in a state where the wearer P wears the lighting device 1, the casing 11 which is reduced in size as much as possible is used (the casing 11 is used, which has substantially a same shape as the support portion 3, and which is formed into an integral body with the support portion 3).

To be more specific, it is sufficient for the lighting device of this embodiment to have specifications where a distance (also referred to as a "treatment distance") L from an irradiation position (light emitting part of the light source) to the affected part is 200 to 400 mm, a light spot having a light field diameter (effective light field diameter) φ of approximately 80 to 120 mm is formed, and maximum illuminance of 15000 to 40000 lux within a range of the light spot is ensured (approximately 20000 to 30000 lux is preferable since it is pointed out that excessively high illuminance may cause the wearer to have eye fatigue). Further, in a case where the light source using LEDs is employed, adopting an arrangement mode of an optical system described later allows the casing 11 to have a reduced size.

To be more specific, the casing 11 having the above-mentioned substantially cylindrical shape is configured as follows. The length of the casing 11 in the lateral direction is smaller than a length of the support portion 3 in the lateral direction. A height H of the casing 11 is lower than a height H1 of the transmission member 30. Further, an amount of protrusion L1 of the casing 11 in a forward direction from a front end edge 3a of the support portion 3 is set to 25 mm or less. Even the casing 11 having such a size can satisfy the above-mentioned specifications. In this case, the light source unit 10 is fixed to an upper end surface of the support portion 3 with screws 6 or the like, and the light source unit 10 is supported between a pair of plate-shaped protruding pieces 7 which protrudes forward. Accordingly, the casing 11 supported between the pair of protruding pieces 7, 7 is in a state of being sufficiently separated from the forehead of the wearer P and hence, there is no possibility of the wearer P feeling heat generated from the light emitting part. Heat radiating fins 11a may be integrally formed on a back surface side of the casing 11.

It is preferable that the casing 11 be supported in a rotatable manner in the upward and downward directions with respect to the protruding pieces 7. With such a configuration, a light field can be moved in the upward and downward directions. A general structure is applicable to a rotation mechanism so that the detailed description of a detailed structure of the rotation mechanism is omitted. To allow an assistant other than the wearer to be able to easily manipulate the rotation mechanism, it is preferable to adopt a configuration that a manipulation lever (not shown in the drawing) is provided to the rotation mechanism so as to allow the rotation mechanism to be rotated in the upward and downward directions.

Next, an arrangement mode of the light source disposed in the casing 11, and the paths of light emitted from the light source are described.

A light source 12 in this embodiment is disposed in the casing. The light source 12 includes an LED board (not shown in the drawing) and condensing lenses 13. LED elements are mounted on the LED board. The condensing lenses 13 cause a predetermined position (the affected part of the patient) to be irradiated with LED light radiated from the LED elements.

The condensing lenses carry out a function to condense light emitted from the LED elements and to irradiate the affected part. Light may be condensed by one condensing lens. However, as described above, by taking into account specificity that the condensing lenses cause light to be condensed with high illuminance at a position with a fixed treatment distance and within a range of a fixed light field diameter, it is desirable to adopt a configuration that respective LED light radiated from a plurality of LED elements is condensed, a plurality of condensing lenses are disposed corresponding to the respective LED elements, and light from the respective condensing lenses is condensed.

To be more specific, in the case of the casing 11 having the above-mentioned substantially cylindrical shape, individually disposing a plurality of condensing lenses along a longitudinal direction of the casing 11 allows light to be efficiently condensed at the position with the fixed treatment distance. Particularly, as shown in Figure 1, when the condensing lenses are disposed at left-right symmetrical positions with respect to a center X of the support portion 3, light can be easily and efficiently condensed on an extension which extends forward at a center position between eyes (at a position which forms a front when the wearer performs visual recognition). In this embodiment, two condensing lenses (condensing lenses 13a, 13b) are disposed on a left side of the center X, and two condensing lenses (condensing lenses 13c, 13d) are disposed on a right side of the center X. Further, one condensing lens 13e is disposed at the position of the center X. In this case, it is sufficient that at least one or more condensing lenses be disposed at each of the left-right symmetrical positions, that is, two or more condensing lenses be disposed in total. When an odd number of condensing lenses are disposed as in a case of this embodiment, the condensing lens may be disposed at the position of the center X.

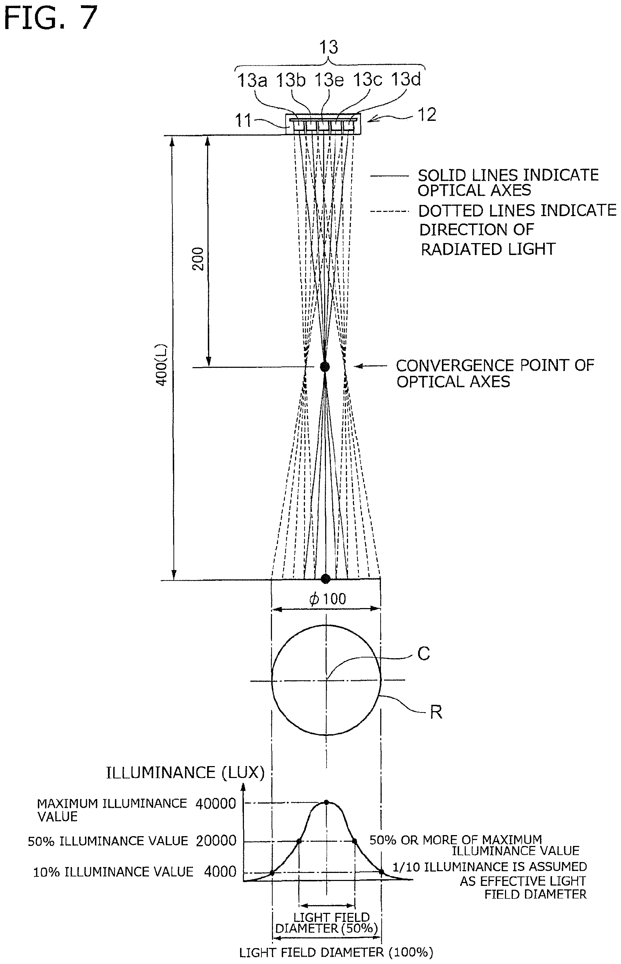

The condensing lenses 13a to 13e shown in Figure 1 have a same configuration. For example, each of the condensing lenses 13a to 13e may be formed of an achromatic lens. One or more LED elements are correspondingly disposed for each condensing lens. The respective condensing lenses are configured such that, as shown in Figure 7, light radiated from the respective condensing lenses forms, with a distance (treatment distance) L of 400 mm ranging from the irradiation position to the affected part, a light spot having the light field diameter (effective light field diameter) φ of approximately 100 mm, and the maximum illuminance within the range of the light spot is 40000 lux. To be more specific, the respective condensing lenses are configured such that the maximum illuminance of 40000 lux can be ensured at a center position C of a fixed light field diameter (diameter of 100 mm), 50% of the maximum illuminance can be ensured at a position 50% of the light field diameter from the center position C, and 10% of the maximum illuminance can be ensured at a position 100% of the light field diameter from the center position C.

Further, by taking into account that various treatment works, such as therapy, is performed while the affected part of the patient is irradiated, the lighting device 1 of this embodiment is configured such that, in generating the light spot with the above-mentioned treatment distance L, respective optical axes (indicated by solid lines) of the respective condensing lenses 13a to 13e converge on one point at a position closer than the treatment distance (in this embodiment, a convergence point, on which the optical axes of the respective condensing lenses converge, is set to a position at an intermediate point of 200 mm, which is approximately half of the treatment distance L).

In this case, even when the convergence point of the optical axes of the respective condensing lenses is set at a position longer than the treatment distance L, it is possible to obtain the light spot having the light field diameter of high illuminance at the position with the treatment distance L. However, in the case where the convergence point is longer than the treatment distance L, when another object, such as a hand, enters between the light source and the treatment position during a therapy work, an influence of a shadow grows (shadow is easily formed). By setting the convergence point of the respective optical axes at a position closer than the treatment distance L as in the case of this embodiment, a region having the highest illuminance is present between the light source and the treatment position. Accordingly, it is possible to reduce an influence of a shadow.

The above-mentioned convergence of the optical axes of the respective condensing lenses 13a to 13e on one point includes not only a mode where the respective optical axes accurately intersect on one point, but also a mode where the respective optical axes converge within an area having a certain diameter. The distance of such a convergence point from the light source is set to an optimum position corresponding to a use purpose (surgical use, oral cavity use, or the like). For each of the respective condensing lenses, it is preferable to use a condensing lens having a focal distance shorter than the above-mentioned distance from the light source to the convergence point. With the use of the condensing lens having such a focal distance, the degree of convergence of light is increased and hence, it is possible to efficiently form the light spot without causing light radiated from the respective condensing lenses and indicated by dotted lines in Fig. 7 to be widely diffused outward in a radial direction.

According to the above-mentioned configuration and arrangement mode of the condensing lenses, lights emitted from the respective condensing lenses are made to overlap with each other at the position with the fixed treatment distance L. Accordingly, it is possible to obtain a region where irradiation light has high illuminance (a light spot region (light field diameter) R where a center C of a region where light from five condensing lenses is made to overlap with each other has the highest illuminance of 40000 lux, a region within a range where the diameter is 50% of the light field diameter has 20000 lux or more, and a region within a range where the diameter is 100% of the light field diameter has 4000 lux or more). In this case, a region where irradiation light from the respective condensing lenses overlaps reduces as a position in the light spot region R is shifted to the outer side in the radial direction from the center C. Accordingly, illuminance does not dramatically vary, but gradually varies (is gradually blurred) as the position is shifted to the outer side. Therefore, a gap between illuminance at the affected part, which is expected to be irradiated, and illuminance of an area around the affected part is reduced and hence, the affected part can be visually recognized easily when a therapy activity is performed.

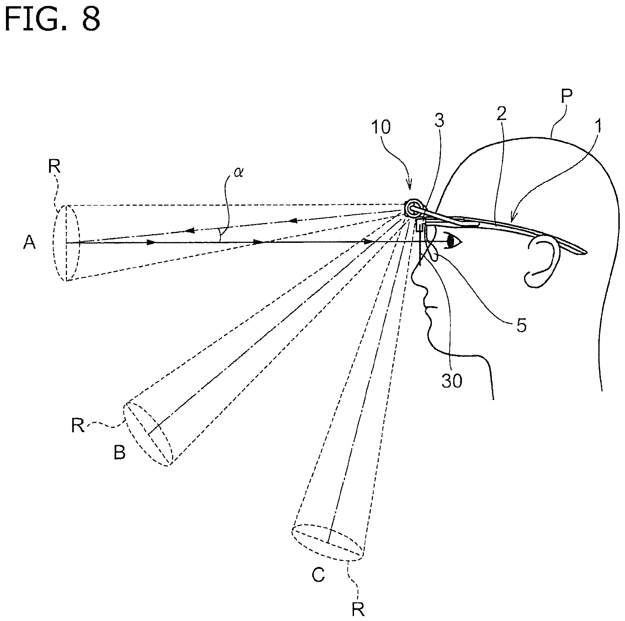

The above-mentioned light source (light source unit 10) and the transmission member 30 are provided in the upward and downward directions of the support portion 3. Particularly, the respective light sources (condensing lenses) are disposed on the support portion 3, disposed above the eyes, along the lateral direction. Accordingly, as shown in Fig. 8, a small elevation angle α is formed between irradiation light from the light source unit 10 and reflected light from the affected part. As described above, the small elevation angle α is formed and hence, in removing glare as described later, reflected light which is reflected in a polarization state can be easily cut, thus enhancing a glare removing effect. Further, the light source unit 10 is supported in a rotatable manner in the upward and downward directions with respect to the support portion 3. Accordingly, even in a case where a wearer moves only a line of sight in frontward and rearward directions without moving the neck, rotating the light source unit 10 can move the light field to a range of the line of sight (see a position A, a position B, and a position C in Fig. 8).

Further, the above-mentioned lighting device 1 of this embodiment is of an eyeglass type. Accordingly, compared with a lighting device where a light source is supported on a mounting band fitted around a head, for example, the lighting device 1 can be easily mounted and removed so that the lighting device 1 has excellent handling and operability.

Power is supplied to the above-mentioned light source unit 10 through a power cord 16 which is introduced from a side end surface of the light source unit, and which is accommodated in the recessed groove 2b formed on the inner side surface of the temple 2. A connection terminal 16a is provided to an end portion of the power cord 16. Connecting a line from an external battery (not shown in the drawing) to the connection terminal 16a allows power to be supplied to the light source unit 10. In this case, a form and arrangement position of the connection terminal 16a, a method for supplying power to the light source unit 10 and the like are not particularly limited. Further, power may be supplied with a method which supplies power from a portable DC battery, or with a method which supplies power from a power source (AC power source) installed in a room, for example.

The lighting device 1 having the above-mentioned configuration is configured to allow the wearer to visually recognize, without generating glare, light emitted from the light source and irradiating the affected part.

Hereinafter, the description is made with respect to a specific configuration (specific configuration which removes glare) for allowing the wearer to visually recognize, without generating glare, light obtained by irradiating the affected part with irradiation light.

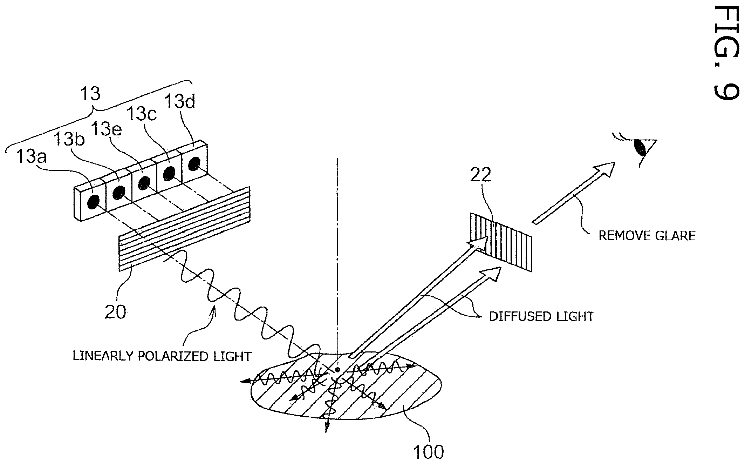

In this embodiment, as shown in a schematic view in Fig. 9, a plate-shaped first polarizing element 20 is mounted on an opening portion formed on a front surface of the casing 11 so as to cover a front surface side of the respective condensing lenses 13a to 13e of the light source unit 10. The plate-shaped first polarizing element 20 converts emitted light which is emitted from the light source to linearly polarized light. By mounting the plate-shaped first polarizing element 20, which extends in the lateral direction, on the opening portion formed on the front surface of the casing 11 as described above, light which transmits through the respective lenses individually disposed in the casing can be integrally changed to linearly polarized light. That is, this first polarizing element 20 converts light (light in a non-polarization state) emitted from the condensing lenses 13a to 13e to linearly polarized light, which has a fixed oscillation direction of an electric field (and a magnetic field), and an affected part 100 is irradiated with this linearly polarized light.

The transmission member 30 which covers a front side of the eyes of the wearer also includes a second polarizing element 22, and the second polarizing element 22 is disposed 90° orthogonal to the polarization axis of the first polarizing element 20.

If the affected part 100, which is an object to be irradiated, is in a mirror surface state, reflected light from the affected part 100 forms linearly polarized light without any conversion. However, unevenness is present on an actual affected part 100 and hence, reflected light forms diffused light (in a non-polarization state) containing components of linearly polarized light. That is, in a case where reflected light from the affected part 100 is diffused and reflected, even if an incident light is linearly polarized light, reflected light of the incident light is brought into a non-polarization state. This light in the non-polarization state contains components of a specularly reflected light (an incident linearly polarized light forms linearly polarized light without any conversion, and is reflected). The components of the specularly reflected light are visually recognized as so-called glossiness (glare) which is the glare of the light source. This glossiness becomes an obstacle when various treatments are performed on the affected part.

The transmission member 30 allows transmission of reflected light from the affected part 100 so as to allow the wearer to visually recognize this transmitted light. As described above, the transmission member 30 includes the second polarizing element 22 disposed 90° orthogonal to the polarization axis of the first polarizing element 20. Accordingly, reflection components (the glare of the light source) which become a cause of glare are removed whereby the wearer can visually recognize only diffused and reflected light with no glare.

Each of the above-mentioned first polarizing element 20 and second polarizing element 22 may be formed of a linearly polarizing filter, a film polarizer, a nano wire grid polarizing plate, an inorganic polarizing plate (a plate obtained such that an aluminum thin film is formed on a glass wafer, and minute slits are formed on the aluminum thin film) or the like, for example. The second polarizing element 22 may be configured such that the transmission member 30 per se is formed as a filter having a polarizing function, or may be configured such that a thin film having the polarizing function is formed on the transmission member 30.

In the above-mentioned configuration, emitted light from the light source is electrically subjected to frequency modulation (pulse modulation) by an external signal to vary phase, amplitude, and a polarization plane, thus accurately aligning an oscillation direction of the electric field of light (an oscillation direction of the electric field of light is brought into an aligned state). Then, the affected part is irradiated with this light through a wire grid polarizing plate forming the first polarizing element having high transmittance. With such configuration, glare can be removed more effectively.

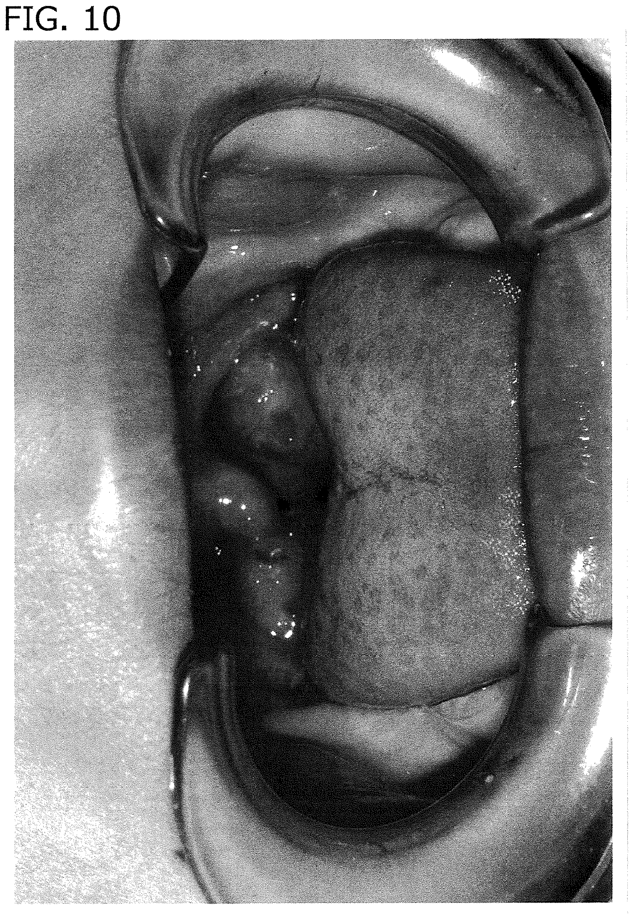

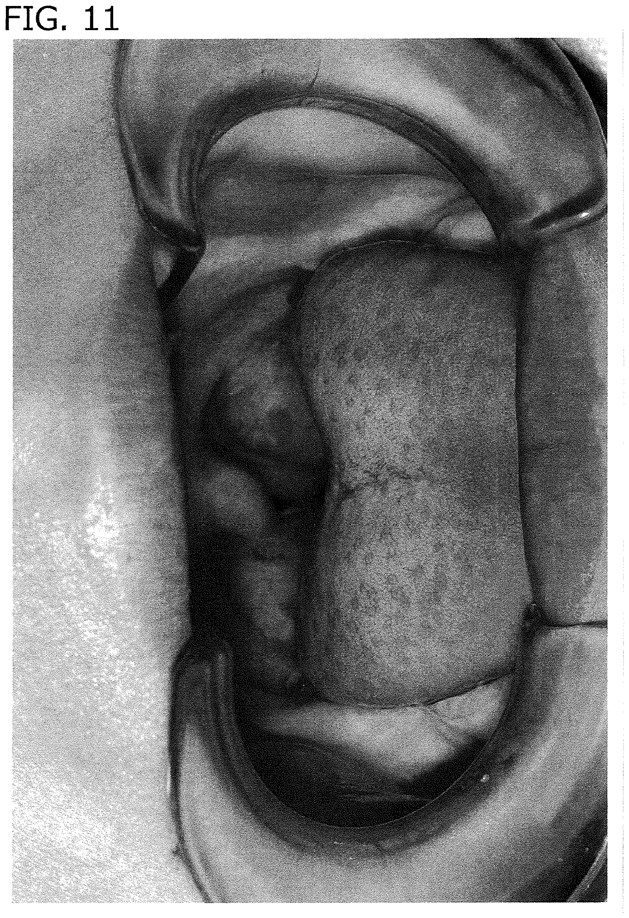

Fig. 10 is a photograph (photocopy of photograph) obtained when a conventional lighting device is used, the photograph showing an inside of an oral cavity where glare is not removed. Fig. 11 is a photograph (photocopy of photograph) obtained when the lighting device 1, provided with optical elements having the configuration shown in Fig. 9, is used, the photograph showing the inside of the oral cavity in a state where glare is removed.

As can be clearly understood from the comparison photograph, providing the first polarizing element 20 and the second polarizing element 22 having the above-mentioned configuration to the light source unit 10 and the transmission member 30 respectively allows the wearer to visually recognize the affected part with no glare. Accordingly, the wearer can obtain correct findings in various operations, examinations, procedure works, or the like and hence, a risk of the patient and the wearer (medical worker) can be reduced, and treatment time and examination time can be shortened whereby a burden on the wearer and the patient can be reduced. Particularly, glare which is reflected off water, body fluid, saliva, oil, or the like of a human body can be effectively removed and hence, the present invention is effective for surgical operations and oral cavity therapy. For example, it becomes possible to rapidly and properly perform a treatment work relating to various medical treatments, such as checking for location of mesenteric blood vessels, a procedure for a bleeding portion of a blood vessel, peeling of a nerve plexus, or a dental implant procedure.

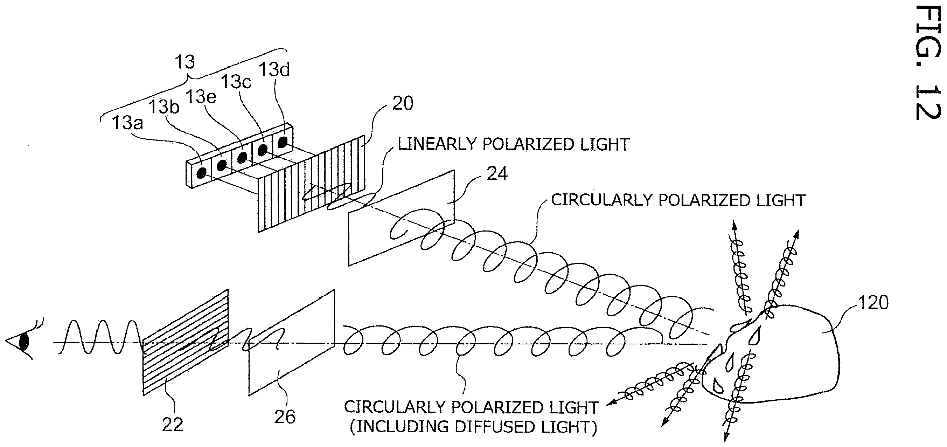

Fig. 12 is a schematic view showing another embodiment of a method for removing glare.

The configuration shown in Fig. 9 illustrates the method for removing glare at the time of allowing the wearer to visually recognize the affected part 100 in a static state. Assume a case where wavelength plates (a 1/2 wavelength plate, a 1/4 wavelength plate, a 1/8 wavelength plate, and the like) are disposed in an overlapping manner in the above-mentioned configuration. In such a case, when water is discharged to an object in a dynamic state, to be more specific, to the affected part, glare caused by reflected light from the water can be removed. For example, during oral cavity therapy for dental implant or the like, a procedure is performed, such as drilling of a tooth, while cooling water is discharged. In such a case, it is also possible to remove glare caused by reflected light from the discharged water.

Hereinafter, a principle for glare removal is described.

In general, in observing a substance in a state of being irradiated with light, when a polarized light where the oscillation direction of the electric field of the light is accurately aligned is used, a polarized light where the oscillation direction of the electric field of the light is accurately aligned is reflected from a subject. In this case, when the affected part is irradiated with circularly polarized light (also including elliptically polarized light) and time required for transmission of the light and variation in intensity of the light are observed, it is possible to obtain information of chirality, which is a difference in a three-dimensional arrangement of molecules.

Usually, a molecule of water reflects or absorbs light corresponding to a wavelength. The molecule of water 120 flowing along the surface of the affected part adheres or flows (jet water stream or the like) along the surface of the affected part so that the surface of the affected part is brought into a state having unevenness. Accordingly, the surface of the affected part cannot specularly reflects light, thus causing scattered reflection. Due to this scattered reflection, color information of the light is lost. The reason the light is viewed hazily (white opaque state) is that the color information of the light is lost, thus preventing the light from being clearly visually recognized. Light has characteristics that when the light is reflected off a water surface or the like, the light is changed to light having large lateral oscillation. Accordingly, when reflected light is circularly polarized, longitudinal oscillation is extremely reduced, and the light is changed to light having oscillation almost only in the lateral direction. Therefore, it is considered that when reflected light is converted to linearly polarized light after being converted to circularly polarized light, glare can be reduced even if an object is in a dynamic state.

As shown in Fig. 12, in the arrangement state of the optical elements shown in Fig. 9, a first wavelength plate 24 is mounted on the first polarizing element 20 in an overlapping manner so that light (in a state of linearly polarized light) which transmits through the first polarizing element 20 is converted to circularly polarized light by the first wavelength plate 24, and the affected part is irradiated with the circularly polarized light (in an actual operation, the water 120 flowing in the dynamic state along the surface of the affected part is irradiated with a light which is the circularly polarized light). Reflected light (in a state of circularly polarized light) of this circularly polarized light is made to be incident on the second polarizing element 22 on which a second wavelength plate 26, having the same configuration, is mounted in an overlapping manner. With such configuration, when the wearer visually recognizes the affected part, the wearer can visually recognize the affected part in a state where specularly reflected light from the water 120 is cut with certainty so that the wearer can visually recognize the affected part in a state where the water is removed from the affected part.

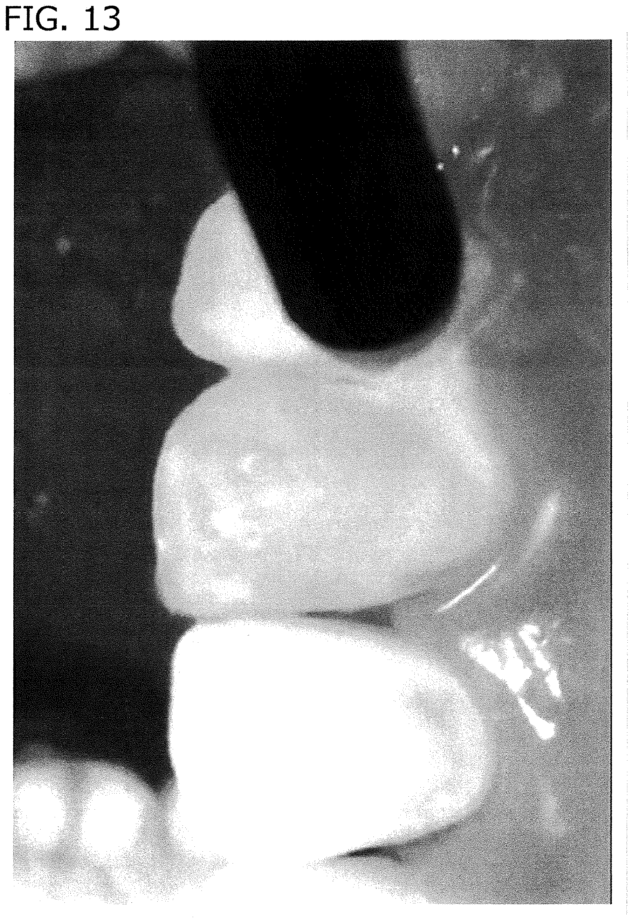

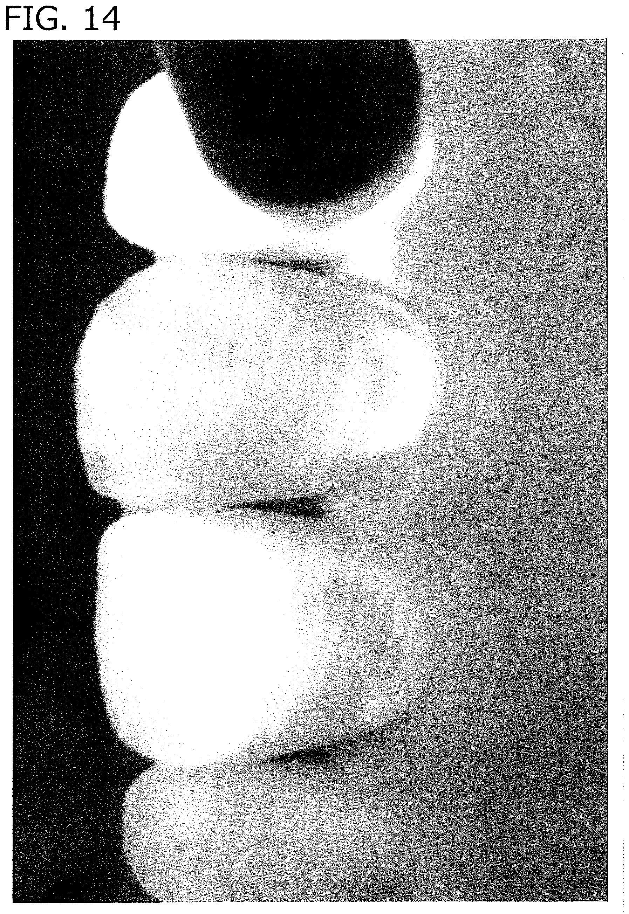

Fig. 13 is a photograph (photocopy of photograph) obtained when the conventional lighting device is used, the photograph showing the inside of the oral cavity where glare is not removed. Fig. 14 is a photograph (photocopy of photograph) obtained when the lighting device 1, provided with the polarizing elements and the wavelength plates having the configuration shown in Fig. 12, is used, the photograph showing the inside of the oral cavity in a state where glare generated due to water in a dynamic state or the like is removed.

As can be clearly understood from the comparison photograph, the above-mentioned first polarizing element 20, on which the first wavelength plate 24 is mounted in an overlapping manner, is provided to the light source unit 10, and the above-mentioned second polarizing element 22, on which the second wavelength plate 26 is mounted in an overlapping manner, is provided to the transmission member 30. With such a configuration, the wearer can visually recognize the affected part in the state where water in a dynamic state is removed. Accordingly, in the same manner as the configuration shown in Fig. 9, the wearer can obtain correct findings in various operations, examinations, procedure works, or the like and hence, a risk of a patient and the wearer (medical worker) can be reduced, and treatment time and examination time can be shortened whereby a burden on the wearer and the patient can be reduced. Glare which is reflected off a liquid body, such as water, body fluid, saliva, or oil of a human body, is removed in addition to glare which is reflected off cooling water discharged toward the affected part. Accordingly, the present invention is also effective for surgical operations or the like in addition to the oral cavity treatment. In an actual treatment, it is also possible to reduce the treatment time to 1/2 or less of conventional treatment time.

As has been described above, light emitted from the light source is polarized (linearly polarized, circularly polarized, or elliptically polarized), and a light reflected from the affected part is subjected to polarization treatment opposite to polarization irradiation of light emitted in front of the wearer. With such a configuration, it becomes possible for the wearer to visually recognize an object in a state where glare is removed or reduced. Accordingly, provided that such a polarization effect can be obtained on a light source unit 10 side and a transmission member 30 side, the specific configuration and arrangement mode of the optical elements can be modified when necessary.

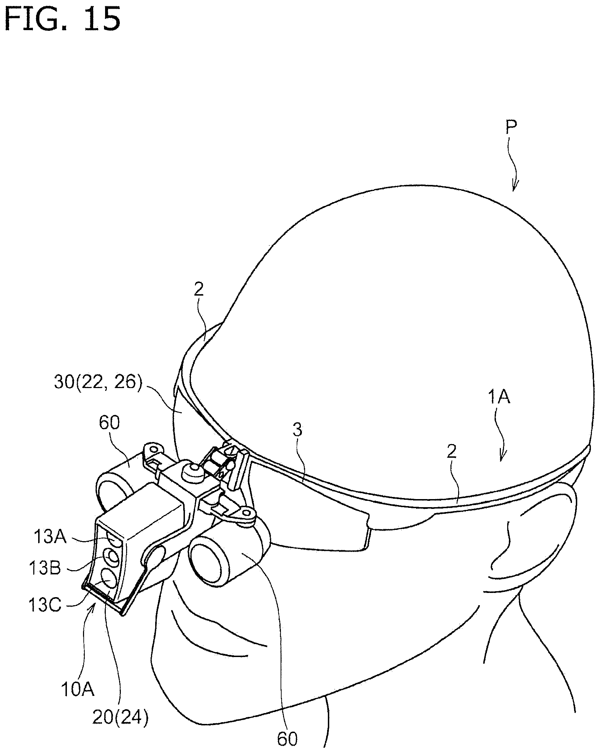

Fig. 15 is a view showing another embodiment of a lighting device according to the present invention.

A lighting device 1A of this embodiment is of an eyeglass type in the same manner as the above-mentioned embodiment. A light source unit 10A is disposed in front of a center portion of a support portion 3, and a binocular loupe 60 is disposed in front of a transmission member 30.

Light sources (condensing lenses 13A, 13B, 13C) of the light source unit 10A are disposed at three portions of the center portion of the support portion 3 along upward and downward directions. In the same manner as the above-mentioned embodiment, the light sources are disposed such that optical axes of the light sources converge on one point. Further, the binocular loupe 60 is a component which allows a wearer to visually recognize a local affected part (a blood vessel or the like at the time of suturing the blood vessel, for example) in the vicinity of the hand of the wearer in an enlarged manner. The binocular loupe 60 shown in the drawing may also be provided to the lighting device 1 of the above-mentioned embodiment.

When the above-mentioned first optical element 20 (and the first wavelength plate 24) is provided to the light source unit 10A, and the above-mentioned second optical element 22 (and the second wavelength plate 26) is provided to the transmission member 30, the lighting device 1A having such a configuration also enables the wearer to visually recognize the affected part in an enlarged manner without glare.

The embodiments of the lighting device according to the present invention have been described heretofore. However, the present invention is not limited to the above-mentioned embodiments, and various modifications are conceivable.

As the lighting device of the above-mentioned embodiment, the eyeglass-type lighting device which can be put on the ears of the wearer has been illustrated. However, the lighting device may have a configuration that the light source unit is supported by a belt, a helmet, or the like mounted on the forehead of the head. Further, the light source unit may be formed of a box-shaped casing, for example, other than a casing formed into the rod shape (cylindrical shape). The arrangement of the light sources may be modified when necessary. The light source may be formed of a component other than an LED element. For example, the light source may be formed of an organic EL, a semiconductor laser light emitting element, or the like. Particularly, if the light source is formed of an organic EL, presence or absence of flaws can be easily determined when an object, such as an industrial product, is visually recognized.

Illuminance and a light field diameter brought about by the light sources can be suitably modified corresponding to the specifications of the light source unit 10, 10A, the number and arrangement mode of light emitting elements, refractive index and arrangement number of the condensing lenses and the like.

Further, the lighting device according to the present invention is also applicable to various industrial fields in addition to a medical field. For example, there is a possibility that a foreign substance, dirt, flaws or the like on a surface of a product may be overlooked if glare is generated. However, mounting the above-mentioned lighting device which can remove glare can prevent production of defective products, overlooking of a defective portion and the like. The lighting device according to the present invention can also be used in various industrial fields, such as various precision machine works, or judging or processing of jewel products.

### EXPLANATIONS OF LETTERS OR NUMERALS

- 1, 1A: LIGHTING DEVICE
- 3: SUPPORT PORTION
- 10, 10A: LIGHT SOURCE UNIT
- 12: LIGHT SOURCE
- 13: (13a to 13e) CONDENSING LENS
- 13A to 13C: CONDENSING LENS
- 20: FIRST POLARIZING ELEMENT
- 22: SECOND POLARIZING ELEMENT
- 24: FIRST WAVELENGTH PLATE
- 26: SECOND WAVELENGTH PLATE
- 30: TRANSMISSION MEMBER
- 60: BINOCULAR LOUPE

## Claims

1. A head-mounted lighting device comprising:
a support portion disposed in front of a forehead of a head of a wearer;
a light source unit provided to the support portion, the light source unit including a light source which irradiates an object with light, and a condensing lens which condenses the light emitted from the light source; and
a transmission member configured to transmit reflected light from the object so as to allow the wearer to visually recognize the reflected light, the transmission member being provided to the support portion and disposed in front of an eye of the wearer, wherein the light source unit includes a first polarizing element and a first wavelength plate, the first polarizing element converting emitted light from the light source to linearly polarized light, the first wavelength plate converting the light transmitted through the first polarizing element to circularly polarized light or elliptically polarized light, and the transmission member includes a second wavelength plate and a second polarizing element, the second wavelength plate having a phase difference equal to a phase difference of the first wavelength plate, the second polarizing element being disposed so as to cause the light transmitted through the second wavelength plate to be 90° orthogonal to a polarization axis of the first polarizing element.

2. The head-mounted lighting device according to claim 1, wherein
the emitted light from the light source is electrically subjected to frequency modulation, and the first polarizing element is formed of a wire grid polarizing plate.

3. The head-mounted lighting device according to claim 1 or 2, wherein
the support portion connects distal end portions of a pair of temples which is put on ears of the wearer, and the light source unit and the transmission member are disposed on upper and lower sides of the support portion respectively.

4. The head-mounted lighting device according to claim 3, wherein
each of the light source unit and the transmission member is supported on the support portion in a rotatable manner in upward and downward directions.

5. The head-mounted lighting device according to claim 3 or 4, wherein
the light source unit has a casing formed into a rod shape so as to extend along the support portion, and two or more condensing lenses are individually disposed in the casing.

6. The head-mounted lighting device according to claim 5, wherein
the first polarizing element is formed of a plate-shaped member which covers a front side of the two or more condensing lenses.

7. The head-mounted lighting device according to claim 5 or 6, wherein
the two or more condensing lenses are supported by the casing such that optical axes of the respective condensing lenses converge on one point.

8. The head-mounted lighting device according to any one of claims 1 to 7, wherein
a binocular loupe is disposed in front of the transmission member.
